(19) Europäisches Patentamt
European Patent Office
Office européen des brevets

(11) Publication number: **0 149 588**
**A2**

(12) # EUROPEAN PATENT APPLICATION

(21) Application number: 85810002.7

(51) Int. Cl.[4]: C 07 C 83/04

(22) Date of filing: 08.01.85

(30) Priority: 13.01.84 GB 8400964
18.05.84 GB 8412684

(43) Date of publication of application:
24.07.85 Bulletin 85/30

(84) Designated Contracting States:
AT BE CH DE FR GB IT LI LU NL SE

(71) Applicant: SANDOZ AG
Lichtstrasse 35
CH-4002 Basel(CH)

(84) Designated Contracting States:
BE CH FR GB IT LI LU NL SE

(71) Applicant: SANDOZ-PATENT-GMBH
Humboldtstrasse 3
D-7850 Lörrach(DE)

(84) Designated Contracting States:
DE

(71) Applicant: SANDOZ-ERFINDUNGEN
Verwaltungsgesellschaft m.b.H.
Brunner Strasse 59
A-1235 Wien(AT)

(84) Designated Contracting States:
AT

(72) Inventor: Strasser, Michael
Bottmingerstrasse 52
CH-4102 Binningen(CH)

(54) **N-(naphthyl-alkyl)-hydroxylamines.**

(57) N-Hydroxy-(5-,6-,7- or 8-alkoxy)-1- or -2-naphthalinalkanamines, e.g. of formula I

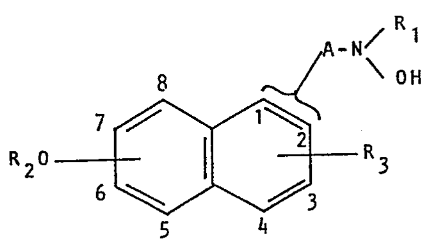

(I)

($R_1$ = hydrogen, $C_{1-8}$alkyl or $C_{4-6}$cycloalkyl, $R_2$ = $C_{1-6}$alkyl, $R_3$ = hydrogen or halogen, and A is $C_{1-6}$alkylene) and their physiologically-hydrolysable and -acceptable esters. The said compounds are lipoxygenase inhibitors.

EP 0 149 588 A2

Case 100-6209

# N-(NAPHTHYL-ALKYL)-HYDROXYLAMINES

The present invention relates to novel N-(naphthyl-alkyl)-hydroxylamines, processes for their production, pharmaceutical compositions comprising them and their use as pharmaceuticals.

Möhrle et al. [Monathsheft für Chemie 108 (2), 273 - 278 (1977); Chem. Ber. 109 (3), 1106 - 1112 (1976); and Arch. Pharm. 314 (10), 836 - 841 (1981)], have described various N-hydroxy-(1-hydroxy)-2-naphthalinmethanamines and N-hydroxy-(2-hydroxy)-1-naphthalinmethanamines and derivatives. Several of these compounds are described as unstable or as having not been isolated as such. No utility for any of these compounds is proposed.

In accordance with the present invention it has now surprisingly been found that both N-hydroxy-1- and -2-naphthalinalkanamines bearing an alkoxy substituent in the 5-, 6-, 7- or 8-position of the naphthalin moiety possess valuable pharmaceutical properties as hereinafter described.

In its broadest aspect the present invention accordingly provides: An N-hydroxy-(5-,6-,7- or 8-alkoxy)-1- or -2-naphthalin-alkanamine or physiologically-hydrolysable and -acceptable ester thereof, in free base or acid addition salt form.

Preferred N-hydroxy-naphthalinalkanamines and esters in accordance with the present invention are compounds of formula I

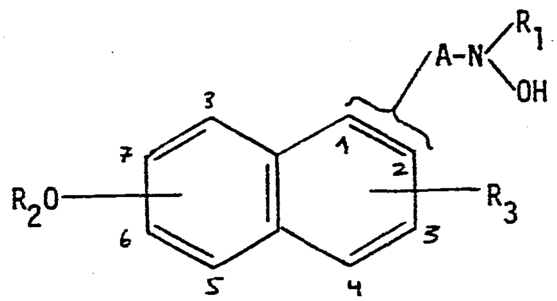

(I)

wherein $R_1$ is hydrogen, $C_{1-8}$alkyl or $C_{4-6}$cycloalkyl,
$R_2$ is $C_{1-6}$alkyl,
$R_3$ is hydrogen or halogen and
A is $C_{1-6}$alkylene,
and the physiologically-hydrolysable and -acceptable esters thereof.

[In accordance with conventional structural representation, in formula I the group $-A-N(R_1)OH$ may occupy the 1- or 2-position only, $R_3$ may occupy the 1-,2-,3- or 4-position only and the group $R_2O-$ may occupy the 5-,6-,7- or 8-position only].

Alkyl groups as $R_1$ and $R_2$ may be branched or straight-chain. In the case of $R_2$ straight-chain alkyl groups are preferred.

When $R_1$ is alkyl it is preferably $C_{1-4}$alkyl, especially methyl. When $R_1$ is cycloalkyl it may be e.g. cyclohexyl.

$R_2$ is preferably $C_{3-5}$alkyl, especially n-propyl.

By halogen is meant chlorine, fluorine or bromine. Preferably $R_3$ is hydrogen.

Alkylene moieties A may be branched or straight chain. Compounds of formula I wherein A is branched chain are of particular interest, e.g. for the treatment or prophylaxis of asthma or inflammation as hereinafter described, especially where e.g. i.v. administration is intended. Otherwise compounds of formula I wherein A is straight chain will generally be preferred, in particular for use in the treatment of psoriasis as hereinafter described. Most preferably A is $C_{1-3}$alkylene, especially $-(CH_2)_2-$ or $-(CH_2)_3-$, most preferably $-(CH_2)_2-$.

The group $R_2O-$ is suitably in the 6-,7- or 8-position. In one group of compounds, when the group $-A-N(R_1)OH$ is in the 1-position, the group $R_2O-$ is in the 6- or 7-position. In a second group, when the group $-A-N(R_1)OH$ is in the 2-position, the group $R_2O-$ is in the 7-position.

A group of compounds of formula I are those wherein $R_1$ is hydrogen or $C_{1-6}$alkyl.

By the term "physiologically-hydrolysable and -acceptable ester" as applied to the N-hydroxy-naphthalinalkanamines of the invention, e.g. the compounds of formula I, is meant esters in which the N-hydroxy group is esterified, e.g. acylated, and which are hydrolysable under physiological conditions to yield an acid which is itself physiologically acceptable, e.g. non-toxic, at desired dosage levels. Such esters include e.g. esters with carboxylic acids, for example $C_{1-4}$alkyl-carboxylic acids, such as the acetic and propionic acid esters.

The present invention also provides a process for the production of an N-hydroxy-(5-,6-,7- or 8-alkoxy)-1- or -2-naphthalin-

alkanamine, for example a compound of formula I as defined above, or physiologically-hydrolysable and -acceptable ester thereof, in free base or acid addition salt form, which process comprises:

a) reacting a (5-,6-,7- or 8-alkoxy)-1- or -2-naphthalinalkane in which the alkane moiety bears a leaving group, for example a compound of formula II

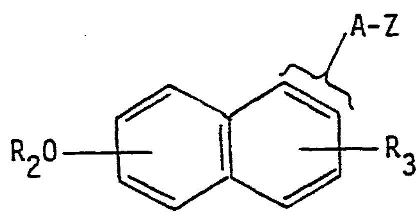

(II)

wherein Z is a leaving group and A, $R_2$ and $R_3$ have the meanings given above, with hydroxylamine or an appropriately N-monosubstituted hydroxylamine, for example a compound of formula III

$HO-NH-R_1$ (III)

wherein $R_1$ has the meaning given above;

b) optionally transforming an N-hydroxy-(5-,6-,7- or 8-alkoxy)-1- or -2-naphthalin-alkanamine obtained in accordance with step a) into another such naphthalinalkanamine, for example alkylating or cycloalkylating a compound of formula I thus obtained wherein $R_1$ is hydrogen to obtain a corresponding compound wherein $R_1$ is $C_{1-8}$alkyl or $C_{4-6}$cycloalkyl;

c) when required, converting an N-hydroxy-(5-,6-,7- or 8-alkoxy)--1- or -2-naphthalinalkanamine, for example a compound of formula I as defined above, obtained in accordance with step a) or step b), into a physiologically-hydrolysable and -acceptable ester thereof; and

d) recovering the N-hydroxy-(5-,6-,7- or 8-alkoxy)-1- or -2-naphthalinalkanamine, for example compound of formula I, or physiologically-hydrolysable and -acceptable estere thereof thus obtained, as the free base or as an acid addition salt thereof.

Process step a) above may be carried out in accordance with means known in the art, for example by reaction of II with III in the presence of an inert solvent or diluent at a temperature of from 0 to 100°C. Suitable leaving groups, e.g. as Z are, for example, halogen atoms, in particular chlorine or bromine atoms, or the methylsulfonyloxy group. Conventiently the reaction is carried out in the presence of a base such as, e.g. pyridine, tri-ethylamine, or sodium or potassium carbonate.

Transformation step b) may be carried out in accordance with any appropriate technique known in the art, e.g. by conventional alkylation e.g. reaction with an appropriate alkyl-halide.

Step c) may also be carried out in accordance with any of the techniques known in the art for the esterification of hydroxylamines, for example by reaction with an appropriate acylhalide, e.g. acylchloride, in the presence of an acid binding agent, at temperatures of from -10 to 50°C, e.g. as hereinafter described in example 30.

Compounds of the invention may be recovered from the initially obtained reaction mixture as such, i.e. in free base form or in

acid addition salt form. Suitable pharmaceutically acceptable acid addition salts, e.g. for pharmaceutical use, include both salts with inorganic acids, such as the hydrochlorides as well as salts with organic acids.

The starting materials of formula II may be obtained in accordance with known procedures, for example by direct halogenation, e.g. bromination of compounds of formula IV

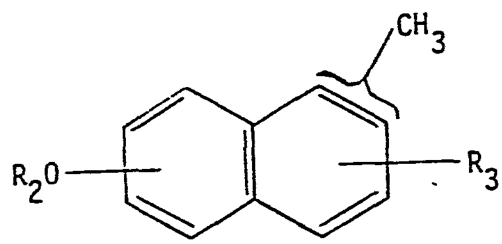

(IV)

to produce compounds of formula II wherein A is $-CH_2-$ and X is halogen or in accordance with the reaction sequence shown below, to produce compounds of formula II wherein A is $C_{2-6}$alkylene and X is methylsulfonyloxy:

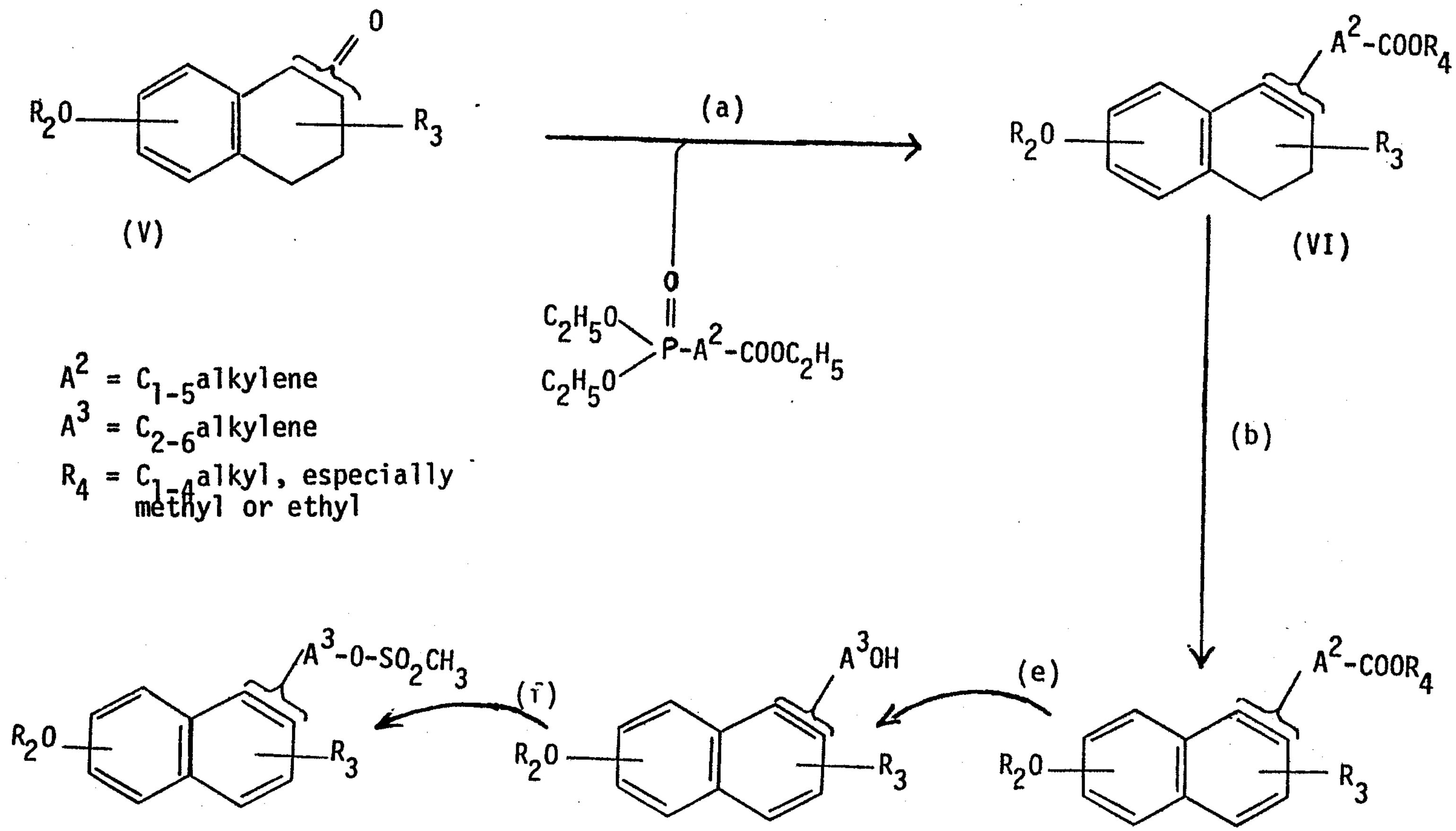


(V)

(VI)

(VII)

(VIII)

(II)

$A^2 = C_{1-5}$alkylene
$A^3 = C_{2-6}$alkylene
$R_4 = C_{1-4}$alkyl, especially methyl or ethyl

Compounds of formulae IV and V are known from the literature or may be prepared analogously to the known compounds. Thus the compound 2-methoxy-7-methyl-naphthalin of formula IV and its preparation are described in Chemical Abstracts 62, 13097 and the compound 7-methoxy-2-tetralone of formula V is commercially available e.g. from the company Aldrich.

Halogenation of compounds of formula IV may be carried out e.g. as hereinafter described in example 23a. Individual reaction steps leading from compounds of formula V, through compounds of formula VIII to II may be carried out e.g. as hereinafter described in examples 1a, 1b, 1e and 1f. $R_2O$- in the compounds of formula V and II may be the same. If desired however, substitution of this group may conveniently be effected between steps (b) and (e), e.g. in accordance with the procedures of examples 1c and 1d. This procedure will be particularly advantageous e.g. in instances where end-products are desired in which the group $R_2O$- is other than methoxy and the corresponding starting material of formula V may be less readily available than the methoxy derivative.

To produce compounds wherein $A^2$ is branched chain alkylene, this may in certain instances be more conveniently effected by derivatisation of a straight-chain alkylene moiety $A^2$ at the formula VII phase in accordance with standard chemical procedures, e.g. in accordance with the methods described in examples 26 and 28.

The following examples are illustrative of the process for the preparation of the compounds of the invention.

EXAMPLE 1

Synthesis of N-hydroxy-N-methyl-(7-propoxy)-2-naphthalinethanamine [Formula I: A = $-(CH_2)_2$-(2-position); $R_1$ = $CH_3$, $R_2O$- = (7) $nC_3H_7O$-, $R_3$ = H].

14 g 7-propoxy-2-naphthalinethyl methanesulfonate, 17 g N-methylhydroxylamine hydrochloride and 40 ml triethylamine in 400 ml abs. ethanol are heated for 18 hours under reflux under an atmosphere of argon. The reaction mixture is evaporated, the residue boiled out 3x with ethyl ether and filtered. The title compound crystallises out on concentration of the combined ether phases: m.p. = 113 - 115°C.

The starting material for the above process is obtained as follows:

1a. 3,4-Dihydro-7-methoxy-2-naphthalin acetic acid ethyl ester [Formula VI: $A^2$ = $-CH_2-$; $R_2O$- = (7) $CH_3O$-; $R_3$ = H; $R_4$ = $C_2H_5$]:

11 g sodium are added over a period of 3 hrs. to 320 ml ethanol under an argon atmosphere. After cooling to room temperature 52.4 g phosphonacetic acid triethyl ester are added. The mixture is again cooled to room temperature, 41.2 g 7-methoxy-2-tetralone are added and the reaction mixture is stirred for ca. 12 hrs. at room temperature. The residue is taken up in ethyl ether, shaken out with $H_2O$, shaken with IN HCl and $NaHCO_3$ solution, dried over $MgSO_4$ and evaporated, and the obtained dark red oil distilled under vacuum. The title compound distills off at 155 - 160°/0.1 mm Hg.

1b. 7-Methoxy-2-naphthalin acetic acid ethyl ester:

43.6 g of the product of step 1a. and 70 ml decahydronaphthalene are stirred for 18 hours at 200° with 2 g 10 % palladium/charcoal. The reaction mixture is cooled to room temperature, filtered over Hyflo and washed with ethanol and methylene chloride. The filtrate is evaporated, the solid residue slurried in hexane and filtered. The residue is retained. The filtrate is distilled and the residue taken up in ethyl ether, crystallised by evaporation and the obtained crystals combined with the retained residue. The title compound is obtained on re-crystallisation from hexane: m.p. = 65 - 68°C.

1c. 7-Hydroxy-2-naphthalin acetic acid ethyl ester:

55 ml borontribromide are added over 15 mins. to a solution of 70 g of the product of step 1b. in 500 ml methylene chloride precooled to -10°C and under an inert atmosphere. The reaction mixture is stirred for 1.5 hrs. at -10°C, poured onto ice/water and the obtained suspension filtered. The residue is washed with $H_2O$ and dried to yield the title compound: m.p. = 133 - 135°C.

1d. 7-Propoxy-2-naphthalin acetic acid ethyl ester:

21 g of the product of step 1c. are added to 27 g 1-bromopropane, 10 ml hexametapol, 25 g dry $K_2CO_3$ and 190 ml di-isopropylketone under an inert atmosphere and the mixture is stirred for 10 hours at 130°C. The obtained suspension is cooled and filtered, the residue washed out with acetone and the filtrate evaporated. The remaining oil, taken up in 300 ml ethyl ether, is shaken 3x each time using 150 ml 0.5 N NaOH and subsequently with 200 ml brine. The etheric phase is dried over $MgSO_4$ and concentrated by evapo-

ration. The title compound is obtained as an oil following chromatographic purification on silica gel.

1e. 7-Propoxy-2-naphthalin ethanol

20 g of the product of step 1d. dissolved in 100 ml tetrahydrofuran are added over 15 minutes to 6.2 g lithium aluminium hydride in 400 ml tetrahydrofuran. The temperature rises to 45°C. The reaction mixture is allowed to stand until it returns to room temperature and is then stirred for 1 hr. 50 ml $H_2O$ are then added drop-wise with caution. A light yellow suspension forms which is then stirred for 30 mins. The obtained suspension is filtered over Hyflo, the residue boiled out with 2 portions of tetrahydrofuran and the combined tetrahydrofuran phases are concentrated by evaporation. The title compound crystallises out on addition of hexane and is re-crystallised from ethyl ether/hexane: m.p. = 97 - 99°C.

1f. 7-Propoxy-2-naphthalinethyl methanesulfonate:

10 ml methanesulfonyl chloride are added drop-wise over 15 mins. to 10 g of the product of step 1e. in 100 ml pyridine pre-cooled to 0°C. The obtained suspension is stirred for 2 hrs. at 0°C and poured onto 100 ml $H_2O$ whereupon the title compound crystallises out. The crystalls are filtered off and dried: m.p. = 93 - 95°C.

EXAMPLES 2 to 22

The following compounds of formula Ia and Ib are prepared analogously to example 1 above.

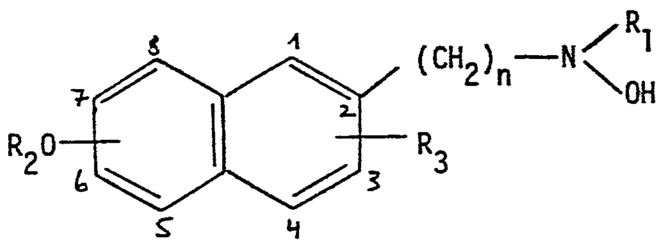

(Ia)

| EXAMPLE | $R_1$ | $R_2O$ | $R_3$ | n | m.p. (°C) |
|---|---|---|---|---|---|
| 2 | H | (7) $nC_3H_7O-$ | H | 2 | 131 - 133 |
| 3 | $CH_3-$ | (7) $nC_6H_{13}O-$ | H | 2 | 170 - 172 |
| 4 | H | (7) $nC_6H_{13}O-$ | H | 2 | 159 - 160 |
| 5 | $CH_3-$ | (7) $CH_3O-$ | (3)Br | 3 | 106 - 109 |
| 6 | $CH_3-$ | (7) $CH_3O-$ | H | 3 | 121 - 124 |
| 7 | $CH_3$ | (7) $C_2H_5O-$ | H | 2 | 115 - 117 |
| 8 | $CH_3$ | (7) $nC_4H_9O-$ | H | 2 | 103 - 105 |
| 9 | H | (7) $nC_3H_7O-$ | H | 2 | 114 - 115 |
| 10 | $iC_4H_9-$ | (7) $nC_3H_7O-$ | H | 2 | 82 - 83 |

| EXAMPLE | $R_1$ | $R_2O$ | $R_3$ | n | m.p. (°C) |
|---|---|---|---|---|---|
| 11 | $CH_3$- | (6) $nC_5H_{11}O$- | H | 2 | 110 - 112 |
| 12 | $C_2H_5$- | (6) $nC_3H_7O$- | H | 2 | 93 - 95 |
| 13 | $CH_3$- | (6) $nC_3H_7O$- | H | 2 | 115 - 117 |
| 14 | $CH_3$ | (8) $nC_4H_9O$- | H | 2 | 109 - 111 |
| 15 | $C_2H_5$- | (8) $nC_4H_9O$- | H | 2 | 83 - 85 |
| 16 | $CH_3$- | (8) $nC_3H_7O$- | H | 2 | 93 - 95 |

M.P. for compounds 3 and 4 is for the HCl salt.
M.P. all other compounds is for the free base.

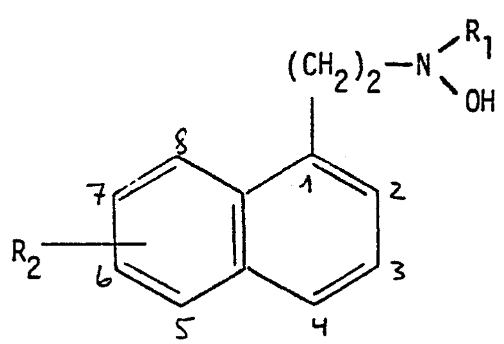

(Ib)

| EXAMPLE | $R_1$ | $R_2O$- | m.p. (°C) |
|---|---|---|---|
| 17 | $CH_3$- | (6) $nC_3H_7O$- | 98 - 100 |
| 18 | $CH_3$- | (6) $nC_4H_9O$- | 79 - 81 |
| 19 | $CH_3$- | (8) $nC_3H_7O$- | 85 - 87 |
| 20 | $nC_3H_7$- | (8) $nC_3H_7O$- | 57 - 59 |
| 21 | H | (7) $nC_3H_7O$- | 56 - 59 |
| 22 | $CH_3$ | (7) $nC_3H_7O$- | 103 - 104 |

M.P. all compounds 17 through 22 is for the free base.

EXAMPLE 23

Synthesis of N-hydroxy-N-methyl-(7-propoxy)-2-naphthalin-methanamine [Formula I: A = $-CH_2-$(2-position); $R_1$ = $CH_3$, $R_2O$- = (7) $nC_3H_7O$-, $R_3$ = H].

2.1 g 7-propoxy-2-naphthalinmethylbromide, 3.1 g N-methylhydroxylamine hydrochloride and 3 ml triethylamine in 30 ml ethanol are heated for 16 hours under reflux in an argon atmosphere. The obtained reaction mixture is evaporated the residue taken up in ethyl ether and the combined ether phases concentrated whereupon the title compound crystallises out: m.p. = 105 - 108°C.

The starting material for the above process is obtained as follows:

23a 7-Propoxy-2-naphthalinmethylbromide [Formula II: $R_2O-$ = (7) $nC_3H_7O-$; $R_3$ = H; A = $-CH_2-$; Z = Br].

22 g 7-methyl-2-propoxy-naphthalin, 20 g freshly recrystallised N-bromosuccinimide, 1.5 g potassium carbonate and 0.5 g dibenzoylperoxide in 750 ml $CCl_4$ are stirred for 16 hours under reflux with UV irradiation under an atmosphere of argon. The obtained brown suspension is filtered, the filtrate evaporated and purified chromatographically on silica gel using hexane/ehtyl ether (95 : 5) as eluant: m.p. 85 - 88°C.

EXAMPLE 24

The compound: N-hydroxy-N-methyl-(7-pentoxy)-2-naphthalinmethanamine [Formula: A = $-CH_2-$(2-position); $R_1$ = $CH_3$; $R_2O-$ = (7) $nC_5H_{11}O-$; $R_3$ = H] is prepared analogously to example 23; m.p. = 68 - 71°C.

EXAMPLE 25

The compound: N-hydroxy-N-methyl-(7-butoxy)-2-naphthalinmethanamine [Formula: A = $-CH_2-$(2-position); $R_1$ = $CH_3$; $R_2O-$ = (7) $nC_4H_9O-$; $R_3$ = H] is prepared analogously to example 23: m.p. = 90 - 91°C.

EXAMPLE 26

Synthesis of N-hydroxy-N-methyl-2-(7-methoxy-2-naphthalinyl)-propylamine [Formula I: -A-N< = -CH($CH_3$)-$CH_2$-N< (2-position); $R_1$ = $CH_3$; $R_2O$- = (7) $CH_3O$-; $R_3$ = H]

The title compound is prepared analogously to example 1 starting from 2-(7-methoxy-naphth-2-yl)-n-propyl methanesulfonate: m.p. for the title compound = 91 - 92°C.

The starting material for the above process is obtained as follows:

26a 2-(3,4-Dihydro-7-methoxy-2-naphthalinyl)-propionic acid methyl ester

The title compound is obtained analogously to example 1a starting from 7-methoxy-2-tetralone and 2-phosphonopropionic acid methyl ester.

26b 2-(7-Methoxy-2-naphthalinyl)-propionic acid methyl ester

21 g of the product of example 26a are stirred for 16 hours at 195°C with 1g 10 % Pd/charcoal in 50 ml decahydronaphthalin. The obtained reaction mixture is filtered hot over Hyflo and the solvent evaporated off to yield the title compound. This reacted further without purification.

26c 2-(7-Methoxy-2-naphthalinyl)-propanol

3.5 g of the product of example 26b in 30 ml tetrahydrofuran are added with protective gassing to a suspension of 1.1 g lithium-aluminium-hydride in 90 ml tetrahydrofuran and the obtained

reaction mixture stirred for 2.5 hours at room temperature. 10 ml $H_2O$ are added carefully and the mixture stirred for a further 30 minutes and then filtered. The residue is boiled out 3x employing 100 ml tetrahydrofuran each time and the combined organic phases are evaporated. The product is obtained initially as a pale brown oil which later begins to crystallise. M.P. for the title compound on re-crystallisation from ethyl ether/hexane = 84 - 86°C.

26d 2-(7-Methoxy-2-naphthyl)propyl methanesulfonate

2.2 g of the product of example 26c are dissolved in 22 ml pyridine and cooled to 0°C. 2.2 ml methane sulfonyl chloride are added drop-wise over 5 mins. to yield a dark brown suspension which is then poured into 300 ml $H_2O$. Extraction is effected using diethyl ether and the ether solution is washed with 100 ml 1N HCl, 1 ml $H_2O$, dried over $Mg_2SO_4$, filtered and evaporated. M.P. for the title compound = 90 - 93°C.

EXAMPLE 27

The compound: N-hydroxy-N-methyl-2-(7-propoxy-2-naphthalinyl)-propylamine [Formula I: -A-N< = -CH($CH_3$)-$CH_2$-N< (2-position); $R_1$ = $CH_3$; $R_2$O- = (7) n$C_3H_7$O-; $R_3$ = H] is prepared analogously to example 26: m.p. = 92 - 94°C.

EXAMPLE 28

Synthesis of N-hydroxy-N-methyl-3-(7-methoxy-2-naphthalinyl)-propyl-2-amine [Formula I: -A-N< = -$CH_2$-CH($CH_3$)-N< (2-position); $R_1$ = $CH_3$; $R_2$O- = (7) $CH_3$O-; $R_3$ = H].

The title compound is prepared analogously to example 1 starting from 3-(7-methoxy-2-naphthalinyl)-propyl-2-methanesulfonate: m.p. for the title compound = 115 - 118°C (HCl salt).

The starting material for the above process is prepared as follows:

28a <u>3-(7-Methoxy-2-naphthalinyl)-2-nitro-2-propene</u>

17 g 7-methoxy-2-naphthalinaldehyde and 7.9 g ammonium acetate in 100 ml nitroethane are heated under reflux for 4 hours. The reaction mixture is cooled to room temperature, diluted with 200 ml $CH_2Cl_2$, washed 2x with $H_2O$, dried over $MgSO_4$, filtered and evaporated. The remaining brown oil is purified chromatographically using silica gel and hexane/ethyl ether (4 : 1) as eluant. M.P. for the title compound = 87 - 90°C.

28b <u>(7-Methoxy-2-naphthalinmethyl)-methyl ketone</u>

6 g of the product of example 28b in 250 ml ethanol are warmed to 60°C. 4.2 g raney nickel and 24.7 g sodium hypophosphite in 100 ml $H_2O$ and 124 ml 1N HCl are added portion-wise over 10 mins. The obtained reaction mixture is stirred for 90 mins. at 60°C. The obtained solution, which has a pH of ca. 3, is cooled to room temperature, filtered over Hyflo and washed well with ethanol. The ethanol is removed by evaporation, the residue diluted with 500 ml $H_2O$, filtered and dried to yield the title compound. M.P. = 87 - 89°C.

28c 3-(7-Methoxy-2-naphthalinyl)-2-propanol

0.5 g sodium borohydride in 20 ml $H_2O$ and a catalytic amount of NaOH are added over 10 mins. to 5 g of the product of example 28b in 200 ml methanol. After stirring for 30 mins. at room temperature, 3 ml 20 % $H_2SO_4$ are added thus bringing the pH to 3. The reaction mixture is poured onto water and extracted 4x with methylene chloride. The collected organic extracts are dried over $Mg_2SO_4$, filtered and evaporated to yield the title compound. M.P. = 48 - 52°C.

28d 3-(7-Methoxy-2-naphthalinyl)-propyl-2-methanesulfonate

The title compound is obtained from the product of example 28c, proceeding analogously to example 26d. M.P. = 98 - 100°C.

EXAMPLE 29

The compound: N-hydroxy-N-methyl-3-(7-propoxy-2-naphthalinyl)-propyl-2-amine [Formula I: -A-N< = $-CH_2-CH(CH_3)-N<$ ; $R_1 = CH_3$; $R_2O-$ = (7) $nC_3H_7O-$; $R_3$ = H] is prepared analogously to example 28: m.p. = 93 - 95°C.

EXAMPLE 30

Synthesis of N-acetoxy-N-methyl-(7-propoxy)-2-naphthalinethanamine

10 g of the product of example 1 are added to 4.7 g triethylamine in 200 ml methylenechloride and the obtained solution cooled to 0°C. 3.45 g acetylchloride are added drop-wise over 5 mins., the

temperature allowed to rise to room temperature and the reaction mixture stirred for a further 1 hour. The obtained solution is duluted with 200 ml methylene chloride, washed 1x with saturated $NaHCO_3$ solution, dried over $MgSO_4$, filtered and evaporated to yield the title compound as a yellow oil: RF (silica gel, ethyl ether/methylene chloride 1:1) = 0.58.

The N-hydroxy-naphthalinalkanamines and esters of the invention as well as their pharmaceutically acceptable acid addition salts possess lipoxygenase inhibiting activity as can be shown in standard pharmacological tests for example in accordance with the following assay:

Human neutrophils are isolated from normal donor blood (buffy coats) by dextran sedimentation, hypotonic lysis of contaminating erythrocytes and several washes in saline. 3.5 x $10^7$ cells/ml in PBS are preincubated at 37°C in the presence of test substance or solvent (control) for 5 min. Calcium ionophore A23187 (20 µM) is then added and incubation continued for 2 mins. before addition of [1-$^{14}$C]-arachidonic acid (0.11 mM) and further incubation for 4 mins. The reaction is terminated by addition of 2 vol of methanol followed by centrifugation. The supernatant is acidified to pH 3. The products are extracted into diethyl ether, and are then separated by thin layer chromatography using the organic phase of ethyl acetate: 2,2,4-trimethyl pentane: acetic acid: water (110:50:20:100, v/v) as the solvent system. The radioactive spots are located by radio-scanning and quantified by liquid scintillation counting. (see Bougeat and Samuelsson, Proc. Nat. Acad. Sci. 76, 2148 - 2152, 1979 and Jakschik et al., Prostaglandins 20, 401 - 410, 1980).

Determined $IC_{50}$ values (concentration at which 50 % inhibition is achieved compared with control) for compounds of the invention in the above assay are of the order of from 3 x $10^{-6}$ to 3 x $10^{-7}$ mol/litre.

The N-hydroxy-naphthalinalkanamines and esters of the invention and their pharmaceutically acceptable acid addition salts are

accordingly indicated for use in the treatment of disorders in which products of the lipoxygenase pathway, for example leukotrienes such as leukotriene C play a direct or indirect pathophysiological rôle, for example for the treatment or prophylaxis of asthma (e.g. as bronchospasmolytic agents), for the treatment of inflammation and, in particular, for the treatment of psoriasis.

For the above usages the required dosage will of course vary depending on the particular compound employed, the mode of administration, the particular condition to be treated and the therapy desired. An indicated daily dosage for anti-asthmatic/anti-inflammatory use is in the range of from about 10 to about 100 mg administered once or in divided doses 2 to 4 times a day in unit dosage form comprising e.g. from about 2.5 to about 50 mg of compound of the invention together with a pharmaceutically acceptable diluent or carrier therefor, or in sustained release form.

For use in the treatment of psoriasis, the N-hydroxy-naphthalinalkanamines and esters of the invention and their pharmaceutically acceptable acid addition salts will suitably be applied topically, e.g. to areas of the skin exhibiting psoriatic lesion. For the purposes of topical application they will preferably be put up in a form suitable or adapted for topical application, e.g. in the form of an ointment, gel, cream, lotion or the like or as active agent in a compress, cataplasm, poultice or the like. Suitable compositions for topical application will comprise the active ingredient together with one or more carriers or diluents suitable for dermal application. Such compositions may also include e.g. thickening agents, preserving agents, stabilizing agents (in particular citric acid), or agents to assist skin penetration as known in the art.

The amount of active agent applied will of course depend again on the particular compound chosen as well as, in particular on the severity of the condition. However in general application in amounts of from about 0.01 to about 1.0 e.g. about 0.3 mg/sq.cm., suitably applied at regular intervals e.g. 1, 2 or 3x daily is indicated, and forms for topical application may suitably comprise from about 0.25 to about 5 %, e.g. about 1 % by weight active ingredient. The following is an example for the preparation of a 1 % composition suitable for topical application.

| Ingredient | Quantity (g) |
|---|---|
| Active agent, i.e. compound of formula I or pharmaceutically acceptable acid addition salt thereof | 1.0 |
| Polyethyleneglycol 300 | 64.25 |
| Transcutol® [Diethyleneglycolmonoethylether] | 10.00 |
| Cetiol HE® [c.f. Fiedler: "Lexikon der Hilfstoffe", 2nd Edition p. 228 (1981)] | 5.00 |
| Glycerol | 5.00 |
| $H_2O$ | 2.00 |
| Citric acid | 0.75 |
| Clucel LF ® [Hydroxypropylcellulose] | 12.00 |
| | 100.00 |

The components are compounded in conventional manner to provide a gel suitable for application in an amount of ca. 30 mg (= ca. 0.3 mg active ingredient)/sq.cm..

As will be appreciated where acid addition salts of the subject compounds are to be applied topically these will be salts which are suitable for dermal application (for example the hydrochloride) and the term "pharmaceutically acceptable" in this context is to be interpreted accordingly.

In accordance with the foregoing the present invention also provides:

i) An N-hydroxy-(5-,6-,7- or 8-alkoxy)-1- or -2-naphthalinalkanamine, e.g. a compound of formula I, or physiologically-hydrolysable and -acceptable ester thereof, in free base or acid addition salt form, for use as a pharmaceutical, i.e. for use in therapy, e.g. for use as a lipoxygenase inhibitor, for example for use in the prophylaxis or treatment of asthma, inflammation or psoriasis.

ii) A method for the treatment or prophylaxis of asthma, inflammation or psoriasis in a subject in need of such treatment, which method comprises administering to said subject an effective amount of an N-hydroxy-(5-,6-,7- or 8-alkoxy)-1- or -2-naphthalinalkanamine, e.g. a compound of formula I, or physiologically-hydrolysable and -acceptable ester thereof, in free base or acid addition salt form;
as well as

iii) A pharmaceutical composition comprising an N-hydroxy-(5-,6-,7- or 8-alkoxy)-1- or -2-naphthalinalkanamine, e.g. a compound of formula I, or physiologically-hydrolysable and -acceptable ester thereof, in free base or acid addition salt form, together with pharmaceutically acceptable diluent or carrier therefor.

CLAIMS

1. An N-hydroxy-(5-,6-,7- or 8-alkoxy)-1- or -2-naphthalinalkanamine or physiologically-hydrolysable and -acceptable ester thereof, in free base or acid addition salt form.

2. A compound according to claim 1 of formula I

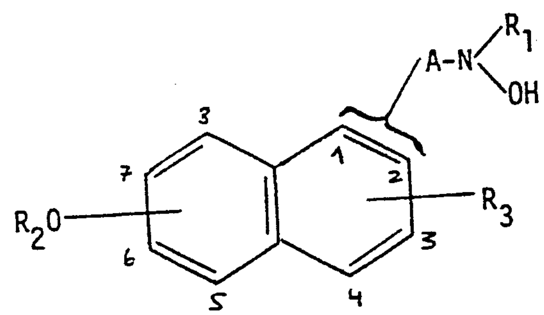

(I)

wherein $R_1$ is hydrogen, $C_{1-8}$alkyl or $C_{4-6}$cycloalkyl,
$R_2$ is $C_{1-6}$alkyl,
$R_3$ is hydrogen or halogen and
A is $C_{1-6}$alkylene,

or physiologically-hydrolysable and -acceptable ester thereof, in free base or acid addition salt form.

3. A compound according to claim 2 of formula I, wherein $R_1$ is hydrogen or $C_{1-6}$alkyl, in free base or acid addition salt form.

4. A compound according to claim 2 which is N-hydroxy-N-methyl-(7-propoxy)-2-naphthalinethanamine in free base or acid addition salt form.

5. A compound according to claim 2 selected from the group consisting of:

N-hydroxy-(7-propoxy)-2-naphthalinethanamine;
N-hydroxy-N-methyl-(7-hexyloxy)-2-naphthalinethanamine;
N-hydroxy-(7-hexyloxy)-2-naphthalinethanamine;
N-hydroxy-N-methyl-(3-bromo-7-methoxy)-2-naphthalinpropanamine;
N-hydroxy-N-methyl-(7-methoxy)-2-naphthalinpropanamine; and
N-hydroxy-N-methyl-(7-propoxy)-2-naphthalinmethanamine;
in free base or acid addition salt form.

6. A compound according to claim 2 selected from the group consisting of:

N-hydroxy-N-methyl-(7-ethoxy)-2-naphthalinethanamine;
N-hydroxy-N-methyl-(7-butoxy)-2-naphthalinethanamine;
N-cyclohexyl-N-hydroxy-(7-propoxy)-2-naphthalinethanamine;
N-hydroxy-N-i.butoxy-(7-propoxy)-2-naphthalinethanamine;
N-hydroxy-N-methyl-(6-pentoxy)-2-naphthalinethanamine;
N-ethyl-N-hydroxy-(6-propoxy)-2-naphthalinethanamine;
N-hydroxy-N-methyl-(6-propoxy)-2-naphthalinethanamine;
N-hydroxy-N-methyl-(8-butoxy)-2-naphthalinethanamine;
N-ethyl-N-hydroxy-(8-butoxy)-2-naphthalinethanamine;
N-hydroxy-N-methyl-(8-propoxy)-2-napthalinethanamine;
N-hydroxy-N-methyl-(6-propoxy)-1-naphthalinethanamine;
N-hydroxy-N-methyl-(6-butoxy)-1-naphthalinethanamine;
N-hydroxy-N-methyl-(8-propoxy)-1-naphthalinethanamine;
N-hydroxy-N-propyl-(8-propoxy)-1-naphthalinethanamine;
N-hydroxy-(7-propoxy)-1-naphthalinethanamine;
N-hydroxy-N-methyl-(7-propoxy)-1-naphthalinethanamine;
N-hydroxy-N-methyl-(7-pentoxy)-2-naphthalinmethanamine;

N-hydroxy-N-methyl-(7-butoxy)-2-naphthalinmethanamine;
N-hydroxy-N-methyl-2-(7-methoxy-2-naphthalinyl)-propylamine;
N-hydroxy-N-methyl-2-(7-propoxy-2-naphthalinyl)-propylamine;
N-hydroxy-N-methyl-3-(7-methoxy-2-naphthalinyl)-propyl-2-amine;
N-hydroxy-N-methyl-3-(7-propoxy)-2-naphthalinyl)-propyl-2-amine;
in free base or acid addition salt form.

7. Process for the production of an N-hydroxy-(5-,6-,7- or 8-alkoxy)-1- or -2-naphthalinalkanamine, for example a compound of formula I as defined in claim 2, or physiologically-hydrolysable and -acceptable ester thereof, in free base or acid addition salt form, which process comprises:

a) reacting a (5-,6-,7- or 8-alkoxy)-1- or -2-naphthalinalkane in which the alkane moiety bears a leaving group, for example a compound of formula II

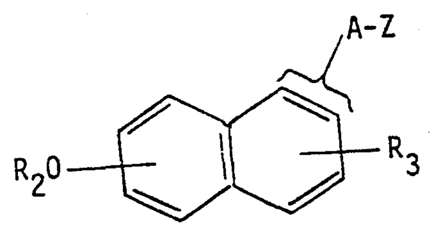

(II)

wherein Z is a leaving group and A, $R_2$ and $R_3$ have the meanings given in claim 2, with hydroxylamine or an appropriately N-monosubstituted hydroxylamine, for example a compound of formula III

$$HO\text{-}NH\text{-}R_1 \qquad (III)$$

wherein $R_1$ has the meaning given in claim 2;

b) when required, transforming an N-hydroxy-(5-,6-,7- or 8-alkoxy)-1- or -2-naphthalinalkanamine thus obtained into another such naphthalinalkanamine, for example alkylating or cycloalkylating a compound of formula I thus obtained wherein $R_1$ is hydrogen to obtain a corresponding compound wherein $R_1$ is $C_{1-8}$alkyl or $C_{4-6}$cycloalkyl; and

c) when required, converting an N-hydroxy-(5-,6-,7- or 8-alkoxy)-1- or -2-naphthalinalkanamine, for example compound of formula I, obtained in accordance with step a) or step b) into a physiologically-hydrolysable and -acceptable ester thereof; and

d) recovering the N-hydroxy-(5-,6-,7- or 8-alkoxy)-1- or -2-naphthalinalkanamine, for example compound of formula I, and physiologically-hydrolysable and -acceptable ester thereof, thus obtained as the free base or as an acid addition salt thereof.

8. A pharmaceutical composition comprising an N-hydroxy-(5-,6-,7- or 8-alkoxy)-1- or -2-naphthalinalkanamine, for example compound of formula I,as defined in any one of claims 2 to 6, or physiologically-hydrolysable and -acceptable ester thereof, in free base or pharmaceutically acceptable acid addition salt form, together with a pharmaceutically acceptable diluent or carrier therefor.

9. An N-hydroxy-(5-,6-,7- or 8-alkoxy)-1- or -2-naphthalinalkanamine, for example compound of formula I,as defined in any one of claims 2 to 6, or physiologically-hydrolysable and -acceptable ester thereof, in free base or pharmaceutically acceptable acid addition salt form, for use as a pharmaceutical, for example for use as a lipoxygenase inhibitor.

10. An N-hydroxy-(5-,6-,7- or 8-alkoxy)-1- or -2-napthalinalkanamine, for example compound of formula I,as defined in any one of claims 2 to 6, or physiologically-hydrolysable and -acceptable ester thereof, in free base or pharmaceutically acceptable acid addition salt form, for use in the prophylaxis or treatment of asthma, inflammation or psoriasis.